# EUROPEAN PATENT SPECIFICATION

(11) **EP 4 321 181 B1**
(45) Date of publication and mention of the grant of the patent: **29.07.2026**
(21) Application number: 22306206.8
(22) Date of filing: 10.08.2022
(51) Int. Cl.: A61L 15/00, A61P 17/02, A61P 31/00, B01D 53/94, A61K 9/00, A61K 9/16, A61K 9/70, B01D 53/02, B01J 20/22, B01J 20/28, B01J 20/30

(54) **NEW ULTRA-MICROPOROUS CRYSTALLINE METAL ORGANIC FRAMEWORKS COMPRISING BISPHOSPHONIC ACID LIGANDS**
NEUE ULTRAMIKROPORÖSE KRISTALLINE METALLORGANISCHE GERÜSTMATERIALIEN MIT BISPHOSPHONSÄURELIGANDEN
NOUVELLES STRUCTURES ORGANOMÉTALLIQUES CRISTALLINES ULTRA-MICROPOREUSES COMPRENANT DES LIGANDS D'ACIDE BISPHOSPHONIQUE

(43) Date of publication of application: 14.02.2024
(73) Proprietor: Centre national de la recherche scientifique, 75016 Paris (FR); École Normale Supérieure, 75005 Paris 5 (FR); Ecole Supérieure de Physique et de Chimie Industrielles de la Ville de Paris, 75005 Paris (FR); Instituto Superior Técnico, 1049-001 Lisboa (PT); Faculdade De Ciências da Universidade de Lisboa, 1749-016 Lisboa (PT)
(72) Inventor: SERRE, Christian, 78370 PLAISIR (FR); TISSOT, Antoine, 94260 FRESNES (FR); CAO, Chenchen, Yongcheng City, Henan Province (CN); LUZIA GONCALVES PINTO, Moisés, 1750-440 Lisboa (PT); SILVA, João, 2700-134 Amadora (PT); ANTUNES, Fernando, 1750-126 Lisboa (PT); PINTO, Rosana, 1500-199 Lisboa (PT)
(74) Representative: Santarelli

(56) References cited:
- WO-A1-2022/160045
- MEDIKONDA PRUDHVIRAJ ET AL: "Adsorption of gases on small-pore aluminum bisphosphonate MOF MIL-91(Al)", JOURNAL OF CHEMICAL SCIENCES, vol. 133, no. 4, 1 December 2021 (2021-12-01), New Delhi, XP093016266, ISSN: 0974-3626, Retrieved from the Internet <URL:https://link.springer.com/content/pdf/10.1007/s12039-021-01964-9.pdf?pdf=button> DOI: 10.1007/s12039-021-01964-9
- TADDEI MARCO ET AL: "Synthesis and Crystal Structure from X-ray Powder Diffraction Data of Two Zirconium Diphosphonates Containing Piperazine Groups", INORGANIC CHEMISTRY, vol. 49, no. 20, 23 September 2010 (2010-09-23), Easton , US, pages 9664 - 9670, XP093016267, ISSN: 0020-1669, Retrieved from the Internet <URL:https://pubs.acs.org/doi/pdf/10.1021/ic1014048> DOI: 10.1021/ic1014048
- CABEZA A ET AL: "From non-porous crystalline to amorphous microporous metal(IV) bisphosphonates", MICROPOROUS AND MESOPOROUS MATERIALS, ELSEVIER, AMSTERDAM ,NL, vol. 114, no. 1-3, 1 September 2008 (2008-09-01), pages 322 - 336, XP022736294, ISSN: 1387-1811, [retrieved on 20080117], DOI: 10.1016/J.MICROMESO.2008.01.018
- MERRILL CRYSTAL A. ET AL: "Pillared Layered Structures Based upon M(III) Ethylene Diphosphonates:? The Synthesis and Crystal Structures of M III (H 2 O)(HO 3 P(CH 2 ) 2 PO 3 ) (M = Fe, Al, Ga)", INORGANIC CHEMISTRY, vol. 44, no. 15, 29 June 2005 (2005-06-29), Easton , US, pages 5273 - 5277, XP093016262, ISSN: 0020-1669, Retrieved from the Internet <URL:https://pubs.acs.org/doi/pdf/10.1021/ic0502269> DOI: 10.1021/ic0502269
- M. MAR GÓMEZ-ALCÁNTARA ET AL: "Synthesis and Characterization of a New Bisphosphonic Acid and Several Metal Hybrids Derivatives", INORGANIC CHEMISTRY, vol. 43, no. 17, 1 August 2004 (2004-08-01), pages 5283 - 5293, XP055066182, ISSN: 0020-1669, DOI: 10.1021/ic049453l
- ZHANG MENGDAN ET AL: "Engineering Metal-Organic Frameworks (MOFs) for Controlled Delivery of Physiological Gaseous Transmitters", NANOMATERIALS, vol. 10, no. 6, 8 June 2020 (2020-06-08), pages 1134, XP093020462, DOI: 10.3390/nano10061134

## Description

### Field of the invention

The present disclosure belongs to the field of nanoporous materials, in particular metal organic frameworks (MOFs) and Lewis-based gas delivery and/or slow release, or the detection of Lewis-based gas(es) in gases or liquid streams.

The present disclosure relates, inter alia, to a new ultra-microporous crystalline metal organic framework solid comprising bisphosphonic acid ligands (also refered to as Phosphonate MOFs), such as MIP-210(M) and uses thereof as a carrier in Lewis-based gas (such as NO) delivery. The disclosure also relates to controlled release of the Lewis-based gas in wounds, for example by topical application. The disclosure also encompasses a synthetic method for producing the new ultra-microporous crystalline metal organic framework solid of the invention.

The Phosphonate MOFs of the present disclosure can be used in various applications such as gas carrier and/or for the controlled release of biologically active gas (NO, H₂S, CO). The Phosphonate MOFs of the present disclosure thus are very versatile and have therapeutic and non therapeutic applications.

The invention is defined by the claims and relates to an ultra-microporous crystalline metal organic framework (MOF)solid for use in the controlled release of the Lewis-based gas in wounds.

The references in square brackets [X] refer to the list of references at the end of the examples.

### Background of the invention

Metal-organic frameworks (MOFs) are a versatile class of porous hybrid crystalline architectures, developed in the last decades, made from the association of inorganic moieties and polycomplexing organic linkers, forming micro- or meso-porous materials whose pore size, shape, surface area and hydrophilic/hydrophobic balance can be tuned for a wide range of potential applications. In the field of adsorption, MOFs can possess active sites, like Lewis, Bronsted or redox or functional polar or apolar groups from the organic linkers, that can interact specifically with polar or quadrupolar molecules, e.g. CO, SOx, NOx or CO₂, to enhance the selectivity towards more inert species (e.g. alkenes).

MOFs have even been used for the controlled release of nitric oxide (NO) but not for delivery over an extended period of time in biological media. There is a need for MOFs having a very efficient and selective capture and/or slow release of Lewis-based gas such as NO.

Iron is one of the cheapest and abundant metal and is an endogenous metal that is highly tolerated by human body and other living organism. Therefore, Fe(III) based MOFs have attracted substantial attention since the early discovery of MOFs. Iron (III) MOFs are particularly attractive for biomedical applications such as drug delivery **[1],** biosensing **[2],** imaging **[3]** and as antimicrobial materials **[4,5]** due to its low toxicity, biodegradability and thus increased biocompatibility. Besides, it is also widely used in environmental remediation with economical, effective, low-toxicity strategies **[6,7].**

They however have two major drawbacks.

First, the diversity of iron (III) MOFs has always been slightly limited, and much focused on polycarboxylate linkers, which is mainly due to the difficulty in obtaining crystalline and pure phase products, with very limited scalable examples reported under green conditions (MIL-88A, functionalized Fe-MIL-53 or MIL-88B(Fe), Fe-MIL-100 and Fe-MIL-127 **[8]**). These compounds can be quite chemically stable but the iron-carboxylate bond is not strong enough in body fluids (e.g. phosphate buffers mimicking blood conditions at pH 7,4) for these MOFs to be used in NO delivery in contact with body fluids.

Second, although some iron-containing MOFs have been synthesized and adopted for biomedical applications, their chemical stability remains unsatisfactory in biological media and consequently they are not fitted for long term delivery applications in contact with these media.

These two difficulties are particularly important in the application of controlled release of biological gases from MOFs **[9].**

MOFs have also been developed for the storage and delivery of different therapeutic gases, known as gasotransmitters **[10,11].** This family consists of CO, H₂S and NO, the latter being the most exploited gas due to the more advanced knowledge about their biological signaling functions. NO is known to bind strongly Lewis acid sites from certain types of MOFs (e.g. MIL-100(Fe)) **[12]** prior to be released when in contact with water (gas or liquid phase). NO is a gasotransmitter that exerts a host of signaling effects on several biological targets and, despite its acute toxicity at high levels, its exogeneous administration at controlled rates (pmol to µmol) proved to be of therapeutic interest for many antithrombotic, antibacterial and wound healing applications **[13].** Currently proposed molecular NO donors have significant drawbacks, particularly those regarding their instability that lead to a non-targeted NO release, along with the codelivery of toxic, carcinogenic and/or pro-inflammatory side products **[14,15].**

Hence, the design of functional solid carriers can be an effective strategy to overcome these limitations by making possible the control over the release kinetics at target sites while securing higher stability to the NO donor with less toxic degradation products.

Nanoporous materials, in particular MOFs, are among the most suitable scaffolds to store and release NO since, besides their tunable composition that allows the development of stable and biocompatible frameworks, they provide a highly efficient packing of NO and the local delivery of pure NO without leaching undesirable species. The NO loading mechanism into these porous materials is typically made by the direct physical sorption or by using Coordinatively Unsaturated metal Sites (CUS) as binding moieties for NO coordination (chemisorption) **[16,17].** The latter allows the tuning of the NO payload by changing the metal or the number of available CUS and ensures stable binding of the NO comparing with the merely physisorbed NO. The release of NO is mainly triggered by contact with water, which replaces the NO on the CUS due to its competitive sorption.

A variety of MOF structures has already been explored, featuring broad storage capacities (1-7 µmol NO mg⁻¹, with the highest value achieved by Ni-CPO-27) **[15,18],** stability in biological media (e.g. MIP-177 with < 9% degradation in 72 hours) **[19],** low toxicity (e.g. Fe-MIL-100, BioMIL-3, MIP-177 **[19-21]**) and suitable release lifetimes (highest duration ca. 2 hours reached by MIP-177). In particular, the document Zhang Mengdan et al., Nanomaterials, 2020, 10, 1134 reviews recent developments in the field of biomedical applications of metal-organic frameworks (MOFs) for the controlled release of physiological gaseous transmitters such as nitric oxide (NO), carbon monoxide (CO), and hydrogen sulfide (H₂S).

When considering using a MOF as a delivery system for medical use, the stability in physiological media and its biocompatibility must be taken in consideration, as they need to remain intact over at least the period they are delivering the therapeutic agent and do not induce any toxicity over the target tissues. Failure in these properties is somehow a problem in MOFs, since they hold organic acids and metals (toxic, in many cases) and are commonly less thermically and moisture stable comparing with inorganic materials (e.g., zeolites) **[12,22].** For instance, CPO-27 (Ni or Co) and HKUST-1 are among the reported porous materials with the highest NO adsorption capacities, but they are not toxicologically ideal once they degrade when exposed to aqueous solutions, leaching the toxic metals present in their structures **[23,24].** On the contrary, iron carboxylate MOFs like MIL-88's, MIL-100 and MIL-127, or endogenous metal(II) MOFs CPO-27(Mg, Zn) do not present toxic impact when in contact with cells **[2,20].** However, they tend to degrade rapidly (hours) in biological media, which compromises the control over the gas release, particularly if a long-term therapeutic delivery is required.

Thus, a MOF that combines both low toxicity and stability in body fluids for several days is crucial to implement in the clinical practice.

The difficulty remains in finding a structure that fits all the requirements along with extended and controlled delivery periods (several hours or days would be preferable) to be therapeutically effective over a wide range of applications.

Therefore, there remains a need for finding stable means for a new class of nanoporous materials, and in particular MOFs, that not only have a good, or even high, stability in biological media but that also show a profile of slow release of Lewis-based gas, and in particular NO, in the same biological media.

### Description of the present invention

To address this need, extensive research have been conducted by the present inventors in order to find a new class of MOFs that are capable to very efficiently and selectively adsorb and control release Lewis-based gases, preferably NO.

The above mentioned drawbacks have been overcome by the new ultra-microporous crystalline metal organic framework solid comprising bisphosphonic acid ligands according to the invention (such as MIP-210(M)), that not only have a high chemical stability but can be synthesized under mild green conditions. The invention as defined by the claims relates to Phosphonate MOFs for use in the controlled release of Lewis-based gases, such as NO.

The inventors successfully synthesized the Phosphonate MOFs according to the disclosure, that perfectly meets the above requirements for NO delivery system and demonstrates an unprecedented controlled release time. The product is obtained in a simple and environmentally friendly (water as solvent), and robust synthesis (not sensitive to reactant ratio, concentration, and even modulator), which is very suitable for industrial large-scale production. Besides, the toxicology of the Phosphonate MOFs according to the invention, such as MIP-210(M) and more particularly MIP-210(Fe), MIP-210(Al) and MIP-210(Ti), is favorable since contain iron, aluminium or titanium as the metal source, which is substantially less toxic when compared to some of the other metals used in MOFs. Thus, the Phosphonate MOFs, such as MIP-210(M) and more particularly MIP-210(Fe), MIP-210(Al) and MIP-210(Ti), fulfil the requirements as a NO delivery system with narrow pore, since they exhibit a porous structure featuring iron, aluminium or titanium metal sites on its inorganic building unit in an ideal position to coordinate NO instead of water, therefore showing a prolonged NO release time in body fluids. After confirming their stability in biological media and low toxicity, the inventors explored the application of the NO loaded MOFs according to the invention, and more particularly MIP-210(M), in which M = Fe, Al or Ti, to promote cellular processes related with wound healing - cell migration and angiogenesis - to demonstrate its potential as NO donor. Other advantages are described in the example section.

Before addressing the description of the disclosure itself, in order to facilitate an understanding of the present disclosure, a number of terms and phrases are defined here:
- As used herein other than the claims, the terms "a," "an," "the," and/or "said" means one or more. As used herein in the claim(s), when used in conjunction with the words "comprise," "comprises" and/or "comprising," the words "a," "an," "the," and/or "said" may mean one or more than one. As used herein and in the claims, the terms "having," "has," "is," "have," "including," "includes," and/or "include" has the same meaning as "comprising," "comprises," and "comprise." As used herein and in the claims "another" may mean at least a second or more. As used herein and in the claims, "about" refers to any inherent measurement error or a rounding of digits for a value (e.g., a measured value, calculated value such as a ratio), and thus the term "about" may be used with any value and/or range.
- The phrase "a combination thereof" "a mixture thereof" and such like following a listing, the use of "and/or" as part of a listing, a listing in a table, the use of "etc" as part of a listing, the phrase "such as," and/or a listing within brackets with "e.g.," or i.e., refers to any combination (e.g., any subset) of a set of listed components, and combinations and/or mixtures of related species and/or embodiments described herein though not directly placed in such a listing are also contemplated. Such related and/or like genera(s), sub-genera(s), specie(s), and/or embodiment(s) described herein are contemplated both in the form of an individual component that may be claimed, as well as a mixture and/or a combination that may be described in the claims as "at least one selected from," "a mixture thereof" and/or "a combination thereof."
- In general, the term "substituted" whether preceded by the term "optionally" or not, and substituents contained in formulae of this invention, refer to the replacement of hydrogen radicals in a given structure with the radical of a specified substituent. When more than one position in any given structure may be substituted with more than one substituent selected from a specified group, the substituent may be either the same or different at every position. As used herein, the term "substituted" is contemplated to include all permissible substituents of organic compounds.
- As used herein, the term "about" can refer to a variation of ±5%, ±10%, ±20%, or ±25%, of the value specified. For example, "about 50" percent can in some embodiments carry a variation from 45 to 55 percent. For integer ranges, the term "about" can include one or two integers greater than and/or less than a recited integer. Unless indicated otherwise herein, the term "about" is intended to include values, e.g., concentration values, proximate to the recited range that are equivalent in terms of the functionality of the individual ingredient, the composition, or the embodiment.
- As used herein, the term "and/or" means any one of the items, any combination of the items, or all of the items with which this term is associated.
- As will be understood by the skilled artisan, all numbers, including those expressing quantities of ingredients, properties such as cavity/pore size and BET specific surface area, reaction conditions, and so forth, are approximations and are understood as being optionally modified in all instances by the term "about." These values can vary depending upon the desired properties sought to be obtained by those skilled in the art utilizing the teachings of the descriptions herein. It is also understood that such values inherently contain variability necessarily resulting from the standard deviations found in their respective testing measurements.
- As will be understood by one skilled in the art, for any and all purposes, particularly in terms of providing a written description, all ranges recited herein also encompass any and all possible subranges and combinations of subranges thereof, as well as the individual values making up the range, particularly integer values. A recited range includes each specific value, integer, decimal, or identity within the range. Any listed range can be easily recognized as sufficiently describing and enabling the same range being broken down into at least equal halves, thirds, quarters, fifths, or tenths. As a non-limiting example, each range discussed herein can be readily broken down into a lower third, middle third and upper third, etc.
- One skilled in the art will also readily recognize that where members are grouped together in a common manner, such as in a Markush group, the invention encompasses not only the entire group listed as a whole, but each member of the group individually and all possible subgroups of the main group. Additionally, for all purposes, the invention encompasses not only the main group, but also the main group absent one or more of the group members. The invention therefore envisages the explicit exclusion of any one or more of members of a recited group. Accordingly, provisos may apply to any of the disclosed categories or embodiments whereby any one or more of the recited elements, species, or embodiments, may be excluded from such categories or embodiments, for example, as used in an explicit negative limitation.
- As used herein, the expression "three-dimensional structure" is understood to mean a three-dimensional sequence or repetition of units or subvariants, as is conventionally understood in the field of MOF materials, that are also characterized as "organometallic polymers".
- As used herein, the term "solid" refers to any type of crystalline material. Said solid may be, for example, in the form of crystals, powder or particles of varied forms, for example of spherical, lamellar, etc. form. The particles may be in the form of nanoparticles.
- MOFs are constructed from bridging organic ligands, also named "linkers" or "linker" or "spacers" or "spacer" that remain intact throughout the synthesis, these ligands acting as linkers in the network of the obtained MOF structure. As used herein, the term "ligand" or "linker" or "spacer" refers to a ligand coordinated to at least two metals, which participates in providing distance between these metals and in forming empty spaces or pores, named also "core" in the MOF. In the present invention, ligand are bisphosphonic acid ligands that are interacting with trivalent cations to form a 3D structure. When included in the MOF structure, the bisphosphonic acid molecule undergoes a partial or full deprotonation and gives rise to a bisphosphonate ion having one, two or three negative charges. The terms "bisphosphonic acid ligand" are thus used to describe the "bisphosphonic acid molecule" that has been included into the MOF structure and had undergone the above described modification.
- As used herein, "Lewis-based gas" refers to a gas that possess an electron pair which is not involved in bonding but may form a dative bond with a Lewis acid to form a Lewis adduct. The gas Lewis-based gas may be chosen from NO, CO and H₂S, preferably NO.
- As used herein, "aliphatic" refers to acyclic or cyclic, saturated or unsaturated carbon compounds, excluding aromatic compounds (IUPAC). Open-chain compounds, whether straight or branched, and which contain no rings of any type, are always aliphatic. Cyclic compounds can be aliphatic if they are not aromatic. Aliphatic compounds can be saturated, joined by single bonds (alkanes), or unsaturated, with double bonds (alkenes) or triple bonds (alkynes). Besides hydrogen, other elements can be bound to the carbon chain, the most common being oxygen, nitrogen, sulfur, and chlorine.
- As used herein, "aromatic" refers to cyclically conjugated molecular entity with a stability (due to delocalization) significantly greater than that of a hypothetical localized structure (e.g. Kekulé structure) that is said to possess aromatic character. Use of the term is based on application of the Hückel (4n + 2) rule and on consideration of the topology of orbital overlap in the transition states (IUPAC).

The present inventors have shown through the present invention, that it is possible to strongly tune the release of Lewis-based gas (preferably NO) in wounds. This is made possible by using the new Phosphonate MOFs of the invention, such as MIP-210(M).

Herein, is reported the development of a new Phosphonate MOFs, such as MIP-210(M) and more particularly iron, aluminium and titanium MOFs - MIP-210(Fe) , MIP-210(Al) and MIP-210(Ti) -, with exceptional slow release due the narrow pores and uncommon Lewis-based gas (preferably NO) adsorption mechanism. The strong interaction between the Lewis-based gas (preferably NO) and the metal sites retards the displacement of the coordinated gas by H₂O once exposed to the blood serum or a phosphate buffer solution at pH 7.4 (mimicking the blood serum) resulting in an extraordinary extended period of NO delivery (over at least 72h), which is by far longer than any other reported material for such application. Besides, MIP-210(Fe), MIP-210(Al) and MIP-210(Ti) have high stability and excellent biocompatibility in biological media compared with other MOFs, which ensure its potential for biomedical applications.

A first object of the invention is an ultra-microporous crystalline metal organic framework solid for use in the controlled release of the Lewis-based gas in wounds, preferably by topical application, the ultra-microporous crystalline metal organic framework solid comprising a three-dimensional succession of units of formula (I):

MₘXₙL•yH₂O (I)

wherein
- M independently represents a metal chosen from Fe, Al, V, Mn, Ti, Zr and mixtures thereof, M being in oxidation state III or IV, preferably a metal chosen from Fe, Al and Ti, and more preferably Fe,
- L represents a bisphosphonic acid ligand,
- X represents an anion, preferably chosen from O²⁻, HO⁻, F⁻, SO₄²⁻, HSO₄⁻ , H₂PO₄⁻, HPO₄²⁻ and PO₄³⁻, preferably chosen from O²⁻ and HO⁻,
- m is an integer from 1 to 4,
- n = 0 or 1, and
- y = 0, 1 or 2

wherein said ultra-microporous crystalline metal organic framework solid is loaded with at least one Lewis-based gas, at least a part of which is coordinated with the metal M, and
wherein the at least one Lewis-based gas is chosen from NO, CO and H2S, preferably NO.

In the present invention, the "ultra-microporous crystalline metal organic framework solid comprising a three-dimensional succession of units of formula (I)" may be addressed as the "Phosphonate MOF" for short.

Advantageously, there can be water inside the pores or lying at the outer surface of the particles. y = 0, if dried completely before gas loading of the MOF.

It is meant by "bisphosphonic acid ligand", a ligand that originate from a bisphosphonic acid molecule (or ligand precursor). When included in the MOF structure the bisphosphonic acid molecule undergoes a partial or full deprotonation and gives rise to a bisphosphonate ion having one, two or three negative charges. The terms "bisphosphonic acid ligand" are thus used to describe the "bisphosphonic acid molecule" that has been included into the MOF structure and had undergone the above described modification

Advantageously, the Phosphonate MOF for use according to the invention may be chosen from the Phosphonate MOFs comprising a three-dimensional succession of units of formula I, in which M represents a metallic center. M may be chosen from Fe, Al, V, Mn, Ti, Zr and mixtures thereof. Fe, Al, Ti and Zr are in oxidation state III. Mn and V may in oxidation state III or IV. Preferably M represents a metal chosen from Fe, Al and Ti, and more preferably Fe.

Advantageously, the Phosphonate MOF for use according to the invention may be chosen from the Phosphonate MOFs comprising a three-dimensional succession of units of formula I, in which X represents an anion. When n = 1, X may be chosen from O₂⁻, HO⁻, F⁻, SO₄²⁻, HSO₄⁻, H₂PO₄⁻, HPO₄²⁻ and PO₄³⁻. X may preferably be chosen from O²⁻ and HO⁻.

Advantageously, the Phosphonate MOF for use according to the invention may be chosen from the Phosphonate MOFs comprising a three-dimensional succession of units of formula I, in which L represents a bisphosphonic acid ligand. The bisphosphonic acid ligand may be chosen from bisphosphonic acid ligands comprising from 4 to 12 carbon atoms. Preferably, the bisphosphonic acid ligand may be chosen from the bisphosphonic acid ligands of formula (II):

HPO₃-CH₂-R-CH₂-PO₃ (II),

in which R is an optionally substituted C₄ to C₆ cyclic or heterocyclic moiety, optionally comprising heteroatoms such as N, optionally aromatic. For instance, R may be a xylylene moiety or a piperazine moiety. More preferably, the ligand L may be chosen from p-xylylenebisphosphonic acid ligand, 1,4-bis(phosphomethyl)piperazine acid ligand, 1,4-bis(phosphomethyl)-2-methylpiperazine acid ligand and mixture thereof.

Advantageously, the Phosphonate MOF for use according to the invention may have an average pore diameter size from 0.3 to 0.5 nm. The average pore size may be measured with the use of the crystallographic information file (CIF) of the said MOF. It may also be calculated from a nitrogen, CO₂, argon or krypton isotherm upon application of mathematical equations such as for instance a BJH (Barret-Joyner-Halenda), a DFT (Density Functional Theory) or a Dubinin-Astakhov method in which an average pore size distribution can be determined, preferably DFT [25].

Advantageously, the Phosphonate MOF for use according to the invention may have a gas loading capacity of 0.5 to 4 mmol of Lewis-based gas per gram of dry MOF, preferably between 1 and 2 mmol/g.

Advantageously, the Phosphonate MOF for use according to the invention may be chosen from the Phosphonate MOFs comprising a three-dimensional succession of units of formula (I) chosen from:
- M(HPO₃-CH₂-C₆H₄-CH₂-PO₃)Xₙ•H₂O,

   M being in oxidation state III or IV,
   with M preferably being Fe, Al or Ti and when M = Al or Fe, n = 0 and when M = Ti, n = 1;
- M(HPO₃-CH₂-C₄H₈N₂-CH₂-PO₃)Xₙ•H₂O,

   M being in oxidation state III or IV,
   with M preferably being Fe, Al or Ti and when M = Al or Fe, n = 0 and when M = Ti, n = 1.

Advantageously, the Phosphonate MOF for use according to the invention may be chosen from the Phosphonate MOFs comprising the following three-dimensional succession units: Fe^{III}(HPO₃-CH₂-C₆H₄-CH₂-PO₃)•H₂O, Ti^{IV}(HPO₃-CH₂-C₆H₄-CH₂-PO₃)OH•H₂O, Al^{III}(HPO₃-CH₂-C₆H₄-CH₂-PO₃)•H₂O, Fe^{III}(HPO₃-CH₂-C₄H₈N₂-CH₂-PO₃)•H₂O Ti^{IV}(HPO₃-CH₂-C₄H₈N₂-CH₂-PO₃)OH•H₂O Al^{III} (HPO₃-CH₂-C₄H₈N₂-CH₂-PO₃)•H₂O.

It is disclosed a Phosphonate MOF for use as a carrier in Lewis-based gas delivery, preferably NO delivery. The delivery may be made via a delivery system. The delivery system may be chosen from a composite polymer, a cream, a scaffold or a hydrogel including the Phosphonate MOFs according to the invention.

In the loaded MOF according to the invention, at least 30% of the adsorbed amount of the Lewis-based gas may coordinate with the metal M.

It is also disclosed a use of a Phosphonate MOF for the decontamination of gaseous streams from Lewis-based gas. Preferably, the Lewis-based gas is NO.

It is also disclosed the use of the Phosphonate MOF for detection of a Lewis-based gas in a gas or in a liquid stream, preferably as a biosensor. Preferably, the Lewis-based gas is NO.

The controlled release of the Lewis-based gas is a slow release. It is meant by "slow release", a release of the Lewis-based gas over a period of at least more than 24 hours, preferably from 24 hours to 72 hours.

The disclosure also relates to other applications related to in vivo delivery of the Lewis-based gas, preferably NO. The disclosure thus also relates to the loaded MOF according to the invention, for use in the controlled release of the Lewis-based gas, preferably NO, in therapeutic applications, preferably in anticancer treatment. The solid MOF according to the disclosure may be used in place of NOonate which are used but with severe side-toxicity effects. The disclosure thus opens up the possibility to deliver Lewis-based gas, preferably NO, inside cells *in vivo* without such toxicity effects.

According to the present disclosure, the Phosphonate MOF is preferably under a form allowing a large exchange surface between the MOF and the environment where the Lewis-based gas may be captured by adsorption. The MOF may for example be in the form of a powder, a granule, a pellet, an extrudate, a monolith, a membrane, a composite embedded in the form of particles, a hydrogel, a film or as a coating. For example, document WO2009/123484 [26] published on October 2009 discloses a useful process for producing polyurethane foam filter material with adsorption capabilities that can be used to support the MOF to carry out the present invention. Other examples are the electropining of polymer containing MOF particles disclosed in documents M. Rose et al. Adv. Eng. Mater. 2011, 13, 356-360 **[27]**, R. Ostermann at al. Chem. Commun. 2011, 47, 442-444 **[28]**, J. Ren et al. Int. J. Hydrogen Energy 2015, 40, 9382-9387 **[29]** and M.R. Khan et al. J. Mater. Eng. Perform. 2016, 25, 1276-1283 **[30]**, that give final composite fiber materials with supported MOF that simplify the application of MOF for the adsorption of volatile organic compounds. Other examples can include the use of MOFs in different shapes as, for example, in the form of granules as previously disclosed in the literature, Valekar et al., RSC Adv, 2017 **[31]** or pellets, Q. Ren, et al.," Chem. Eng. J., , 2015 **[32]**, or by coating specific supports, as disclosed by Gkaniatsou et al., Nano Energy, 2020 **[33]**.

According to the present disclosure, the Phosphonate MOF as powder or the Phosphonate MOF under the form as described in previous paragraph may be comprised in a device, preferably an antimicrobial device.

It is also disclosed an antimicrobial device comprising the loaded MOF according to the invention. The device may be a transdermal patch or a coating for a biomedical device.

It is also disclosed a synthetic method for producing a porous crystalline metal organic framework solid comprising a three-dimensional succession of units corresponding to the following formula (I) as defined above, comprising the steps:
a) introducing a metal source, preferably under the form of a salt, alkoxide, hydroxide or oxide, and a bisphosphonic acid precursor of the ligand L, in a solvent,
b) stirring the mixture obtained in step a) for at least 15 minutes, preferably at least 30 minutes,
c) heating the solution obtained in step b) at a temperature from 30 to 180°C, preferably 100°C, under solvothermal conditions, from 5 to 48 hours, preferably 20 hours
and obtaining the porous crystalline metal organic framework solid, wherein M and L are defined as above.

Advantageously, the metal source may be a salt (such as Fe(NO₃)₃, FeCl₃ or AlCl₃) , an alkoxide (such as Ti(OR)₄, in which R is a C1 to C4 hydrocarbon chain), a hydroxide (such as Fe(OH)₃ or Al(OH)₃) an oxide (such as Fe₂O₃, Al₂O₃ or TiO₂), a sulfate (such as FeSO₄, Fe₂(SO₄)₃, Al₂(SO₄)₃ or TiOSO₄ or Ti(SO₄)₂) or an acetate (such as Fe₃O(Z)(CO₂CH₃)₃ (Z = OH, Cl or ClO₄), Al(CO₂CH₃)₂ or Ti(CO₂CH₃)₂).

Advantageously, the bisphosphonic acid precursor of the ligand L may be chosen from bisphosphonic acid ligand precusors comprising from 4 to 12 carbon atoms. Preferably, the bisphosphonic acid ligand precursor may be chosen from the bisphosphonic acid ligand precursors of formula (II'):

H₂PO₃-CH₂-R-CH₂-PO₃H₂ (II'),

in which R is an optionally substituted C4 to C6 cyclic moiety, optionally comprising heteroatoms such as N, optionally aromatic. For instance, R may be a xylylene moiety or a piperazine moiety. More preferably, the precursor of the ligand L may be chosen from p-xylylenebisphosphonic acid, 1,4-bis(phosphomethyl)piperazine acid, 1,4-bis(phosphomethyl)-2-methylpiperazine acid and mixture thereof.

Advantageously, the solvent is chosen from water, organic solvent(s) or mixtures thereof. The organic solvent(s) may be chosen from ethanol, isopropanol and mixtures thereof.

It is meant by "solvothermal conditions", a synthesis performed in a sealed vessel at a temperature higher than the boiling point of the solvent under autoaeneous pressure.

Advantageously, the method above-disclosed may further comprise a step of washing the MOF with water and/or ethanol.

It is also disclosed a synthetic method for producing a loaded MOF according to the invention comprising a step of contacting the Phosphonate MOF according to the invention with a pure gas chosen from Lewis-based gas, under a pressure from 20 to 100 kPa, preferably 80 kPa, in a closed cell, at a temperature from 15 to 25°C, for 1 to 5 days, preferably 3 days.

The following experiments and results confirms the benefits of the present disclosure over the prior art solutions.

The following representative examples and figures are intended to help illustrate the invention, and are not intended to, nor should they be constructed to, limit the scope of the invention as defined by the claims. Indeed, various modifications of the invention as defined by the claims and many further embodiments thereof, in addition to those shown and described herein, will become apparent to those skilled in the art from the full contents of this document, including the examples which follow and the references to the scientific and patent literature cited herein.

The following examples contain important additional information, examplification and guidance that can be adapted to the practice of this invention as defined by the claims.

### Brief description of the drawings

Figure 1 represents the structure of MIP-210(Fe) viewed (a) along the a-axis and (b) along the b axis. (c) A corner-sharing chain of M₂O₁₂ octahedra. Iron atoms are represented by octahedra, phosphonate groups by tetrahedra and carbon atoms by gray spheres.
Figure 2 shows the PXRD patterns (λ = 1.5406 Å) of MIP-210(Fe) MIP-210(Al) and MIP-210(Ti) compared with the simulated pattern from the single crystal structure.
Figure 3 shows the NO release profile from the MIP-210(Fe) in liquid medium obtained as described in the Example 3.

### Examples

According to the present invention, the Phosphonate MOFs, such as MIP-210(M), and their preparation can be understood further by the examples that illustrate some of the processes by which these materials are prepared or used. It will be appreciated, however, that these examples should not be construed to limit the invention as defined by the claims.

### Example 1: Materials synthesis

All chemicals were purchased from commercial supplier and used as received without further purification. p-xylylenebisphosphonic acid (H₄mbpa), 99%, SIKEmia. Ferric chloride hexahydrate, 98%, Fisher. Ethanol absolute, ≥99%, Acros. MilliQ water, Millipore system, Sodium hydrosulphite (≥82% (RT)) and haemoglobin human lyophilized powder from Sigma-Aldrich, Nitric oxide gas (99.99%), Air Liquide.

For cells culture and respective assays, HeLa cells were obtained from American Type Culture Collection (Manassas, VA, USA), HEKn cells from Thermo Fisher Scientific and HUVEC from Live Technologies. RPMI-1640 without L-glutamine was obtained from Corning Inc.; penicillin-streptomycin, L-glutamine, foetal bovine serum (FBS), trypsin (2.5%, without phenol red), Epilife medium with 60 µM calcium, human keratinocyte growth supplement kit, Medium 200 PRF, low serum growth supplement (LSGS), trypsin-EDTA (0.25% without phenol red), trypsin neutralizer solution and Geltrex^{™} LDEV-Free Reduced Growth Factor Basement Membrane Matrix were all purchased from Thermo Fisher Scientific.

Powder X-ray diffraction (PXRD) data were recorded on a high-throughput Bruker D8 Advance diffractometer working on transmission mode and equipped with a focusing Göbel mirror producing CuKα radiation (λ=1.5418 Å) and a LynxEye detector. Nitrogen adsorption isotherm was collected on a Micromeritics Tristar instrument at 77K. SEM-EDX results were recorded with an FEI Magellan 400 scanning electron microscope. TGA data were collected on Mettler Toledo TGA/DSC 2, STAR System apparatus with a heating rate of 5°C/min under the oxygen or Nitrogen flow. Structural models were further confirmed by single-crystal synchrotron diffraction data collected with micro-focused X-rays on the Proxima 2A beamline (Synchrotron SOLEIL) using a suitable single crystal.

**Synthesis of MIP-210(Fe)** (Fe^{III}(HPO₃-CH₂-C₆H₄-CH₂-PO₃)•H₂O): FeCl₃•6H₂O (150 mg, 0.555 mmol) and H₄mbpa (111 mg, 0.417 mmol) were mixed with 5 mL of water in a 50 mL DURAN clear glass with blue polypropylene screw cap by sonicating for 15 mins and then put the solution into 120°C oven for 20 hours, leading to the formation of MIP-210(Fe). The product was collected by centrifugation, washed with water and ethanol, and dried under air, leading to 120mg of bright yellow powder, which corresponds to a yield of 90% based on ligand (due to the excess of metal salt). The synthesis can be scaled up 5 times without any change of samples quality.

Besides, the same product was obtained using and iron salt to ligand molar ratio ranging from 0.5 to 1.5 under similar conditions. The same product was also obtained by changing the water content from 5 to 10 mL, or adding formic acid or acetic acid in the reaction mixture.

**Synthesis of MIP-210(Al):** AlCl₃•6H₂O (134 mg, 0.555 mmol) and H₄mbpa (111 mg, 0.417 mmol) were mixed under stirring with 5mL of water in a 50 mL round-bottom flask and reacted overnight under reflux, leading to the formation of MIP-210(Al). The solid was then washed with 500 mL of water under reflux and then with 30 mL of acetone. The product was then collected by centrifugation and dried under air. The whole reaction can be scaled up five times, leading to the same reaction product with similar properties.

**Synthesis of MIP-210(Ti):** TiOSO₄ (50 mg, 0.320 mmol) and H₄mbpa (50 mg, 0.188 mmol) were mixed under stirring with 5mL of water in a 50 mL round-bottom flask and reacted overnight under reflux, leading to the formation of MIP-210(Ti). The solid was then washed with water under reflux and then with 30 mL of acetone. The product was then collected by centrifugation and dried under air.

### Example 2: Materials characterization

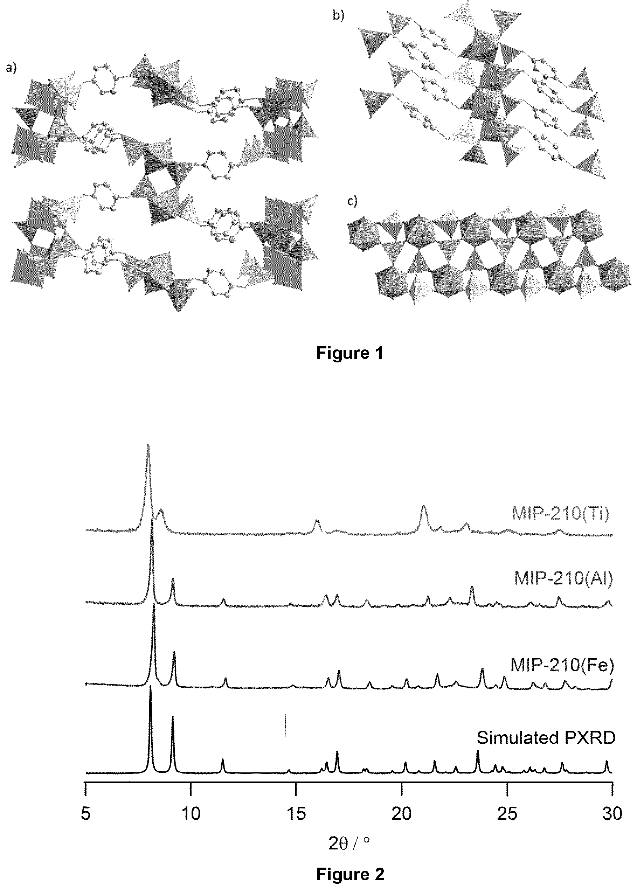

The structure of MIP-210(Fe) was determined by single crystal X-Ray diffraction. The compound crystallizes in a monoclinic space group P2(1)/n with unit cell parameters of a=5.1440Å, b=10.7106Å, c=21.0654Å and β=93.222°, corresponding to a cell volume of 1191.1Å³. In the structure, there is a 1D channel along the a axis, characteristic of a typical bnn topological network. One of the diphosphonate group is connected to two iron atoms and the other one is connected to three iron atoms to form a chain (Figure 1). The simulated PXRD patterns of MIP-210(M) (M=Fe, Al, Ti) matches well with the structure determined from single crystal, indicating that the three compounds are isostrucural and confirming the purity of the three phases (Figure 2). In addition, the thermogravimetric analysis of the MIP-210(M) (M=Fe, Al, Ti) further confirmed the purity of the compounds.

### Exemple 3 : Results

A. Thermal and chemical stability of MIP-210(Fe) was assessed. VT-PXRD results indicate the framework can hold over 300 °C. The chemical stability was evaluated in water, in pH=7.4 PBS solution and in three representative cell growth media (RPMI-1640, Medium 200 and Epilife) at 37 °C. Any degradation was observed after seven days in water and PBS and a minor degradation was detected after 3 days of incubation in biological medium, with no changes in its morphology and cristalinity. This MOF is more stable than other iron MOFs studied for NO adsorption (e.g. MIL-100(Fe) and MIL-127(Fe)). The integrity of the MOF is important to ensure the controlled release application and to avoid the leaching of by-products.

B. Cytotoxicity assessment was performed using three different cell lines: HeLa (immortal cell line derived from cervical cancer cells), HEKn (Human Epidermal Keratinocytes, neonatal) and HUVEC (Human Umbilical Vein Endothelial Cells). MIP-210(Fe) presented a remarkable biocompatibility (>70% viability) at relevant concentrations (between 45-450 µg mL⁻¹) for NO application. This feature together with the high stability in biological environment and to the fact of possessing non-toxic metals in its structure, proves that this MOF is safe to be used in biomedical applications. Most MOFs studied for the same purpose do not combine all these features, either because they possess toxic metals in their frameworks (e.g., CPO-27 (Ni, Co), vitamin B₃ MOFs (Ni, Co)) or because they easily degradates in biological media (e.g. HKUST-1,) which normally, in both cases, raises toxicity.

### C. NO therapeutic release application

A serie of analysis have been carried out to validate the capacity of MIP-210(Fe) to adsorb and release NO in a controlled way:
- DFT calculations;
- Gravimetric NO adsorption;
- FTIR *in situ* NO adsorption/release monitorization; and
- NO release measurments in liquid phase.
- Evaluation of cellular processes related to wound healing

Computational investigation was carried out to predict the adsorption of NO and H₂O. The N-end geometry towards Fe was found to be energetically favoured compared to the O-end and side-on analogues. Two situations can be considered since NO has unpaired electron: NO molecule either aligned parallel to Fe associated with an adsorption energy (E_{ads}) of -27 kJ mol⁻¹ or aligned antiparallel to Fe with E_{ads} =-53 kJ mol⁻¹. This is consistent with the corresponding Fe-N distance (2.91 Å vs 2.37 Å) in the two scenarios. Therefore the N-end adsorption of NO molecule with its spin antiparallel to Fe was considered as the most stable adsorption configuration in following calculations. On the other hand, the interaction of H₂O with the Fe-CUS (O-end adsorption) is even stronger than NO by about 30 kJ mol⁻¹ with E_{ads} of -82 kJ mol⁻¹.

NO adsorption/storage capacity was then investigated using gravimetric measurments. Briefly, MIP-210(Fe) (previously outgassed at 120 °C for 12 hours) was left in contact with 80kPa NO and after 3 days, the material showed an adsorption capacity of 1.86 mmol g⁻¹. The adsorption profile is slow and the plateau was not verified after that period. The amount adsorbed is between the range of the reported MOFs studied for NO application (0.8 - 7 mmol˙g⁻¹) [15], showing lower capacity comparing with those that also have unsaturated Fe centers (i.e. Fe-MIL-100, Fe-MIL-127 and Fe-MIL-88-A with 4.5 mmol˙g⁻¹, 2.2 mmol˙g⁻¹, 2.5 mmol˙g⁻¹ NO adsorption capacities, respectively) **[20,34].**

To perceive the mechanism of NO adsorption in this material, *in situ* FTIR studies with NO present were conducted. IR spectra demonstrate the appearance of a NO adsorption band at 1578 cm⁻¹, which intensity increased with time as consequence of the slow NO adsorption release profile that characterizes this material. This peak corresponds to NO coordinated on Fe sites but with an uncomun configuration, where the angle of the created Fe-N-O bond is shortened to 119 °. This NO adsorption configuration was not observed for any other Fe-based MOFs (i.e. MIL-100, MIL-127, MIL-88) **[20,34]** , where the band assigned to the NO coordinated on Fe sites is ca. 1800 cm⁻¹ and the angle of the formed bond is ca. 180 °. The bond created between MOF-210(Fe) and NO persisted after outgassing, confirming the strong stability of the Fe-NO species formed. This strong chemisorption observed is the most favourable adsorption mechanism for therapeutic delivery, providing a more controlled release and a safer storage of the loaded material.

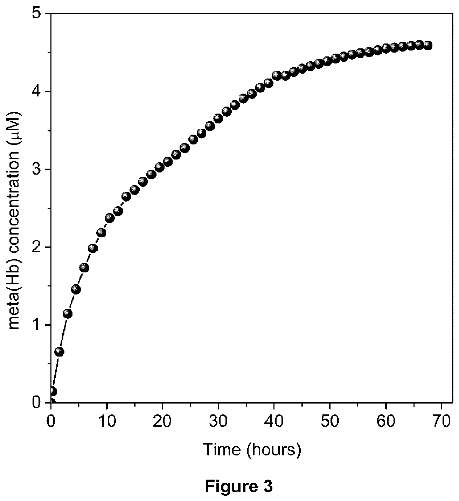

NO release quantification in biological media was carried out using the oxyhemoglobin assay, previously described by Feelish *et al..* **[35]** NO release rate by this MOF is exceptionally slow, ensuring a gradual delivery of NO over at least 70 hours (demonstrated in **Figure 3**). This period may indicate a complete release or a full conversion of the oxyhemoglobin present in the buffer medium in to methemoglobin. Nevertheless, it demonstrates an initial sustained and very slow NO release rate over 70 hours (almost 3 days), never observed for any other reported NO-releasing porous materials, which are limited from several minutes to few hours. For instance, the longest release obtained by this class of materials was 4 hours by a modified ETS-4 with copper incorporated in the structure. **[36]** . In addition to this singular release lifetime, MIP-210(Fe) guarantees a sustained release from the beginning, preventing the initial burst of release characteristic in many NO-delivery strategies. Considering the risk of releasing concentrated NO doses in the biological systems, this MOF is among of the most promising carriers for NO therapeutic delivery, with unique features that allow it to be used in a wide spectrum of applications. To explore such therapeutic potential, first demonstrations were carried out *in vitro* by performing cell migration and angiogenesis assays. Both cellular responses are pivotal for wound healing process and NO plays a signalling role on those processes, contributing to a faster migration and tube formation. The ability of NO released by MIP-210(Fe) to induce HUVEC migration was evaluated by using the Oris^{™} cell migration assay. NO-loaded MIP-210(Fe) at 11.75 µg mL⁻¹ was able to induce a significant (p<0.01) faster HUVEC cells migration (ca. 8%) after 24 hours, comparing with the untreated cells and cells treated with NO-free MIP-210(Fe).

The angiogenic effect of NO released by MIP-210(Fe) was observed through an *in vitro* endothelial tube formation using endothelial cells (HUVEC) and Geltrex^{™} as a 3D extracellular matrix. NO-loaded MIP-210(Fe) treated cells (11.75 µg mL⁻¹) demonstrated a significant (p<0.5) richer network (ca. 25%) in comparison with the cell control and the blank MIP-210(Fe) groups after 18 hours.

Herein, were developed biocompatible ultra-microporous iron(III), aluminium(III) and titanium(III) bisphosphonate MOFs based on a green synthesis for storage and therapeutic release of NO.

The new Phosphonate MOFs, such as MIP-210(M), according to the invention showed an unprecedent very slow and controlled release of NO in biological fluids. This feature permits the safe use of these new donors, without releasing a burst of NO that normally raises toxicity and avoids the frequent replacement of the delivery system usually needed to support the continuous gas release at therapeutic levels. The angiogenic potential of the new compounds was confirmed at the cellular level, giving the first results comforting potential therapeutic applicability.

### LIST OF REFERENCES

**[1]** Horcajada, P.; Serre, C.; Maurin, G.; Ramsahye, N. A.; Balas, F.; Vallet-Regí, M.; Sebban, M.; Taulelle, F.; Férey, G. Flexible Porous Metal-Organic Frameworks for a Controlled Drug Delivery. J. Am. Chem. Soc. 2008, 130 (21), 6774-6780. https://doi.org/10.1021/ja710973k.
**[2]** Ai, L.; Li, L.; Zhang, C.; Fu, J.; Jiang, J. MIL-53(Fe): A Metal-Organic Framework with Intrinsic Peroxidase-Like Catalytic Activity for Colorimetric Biosensing. Chem. - A Eur. J. 2013, 19 (45), 15105-15108. https://doi.org/https://doi.org/10.1002/chem.201303051.
**[3]** Shang, W.; Zeng, C.; Du, Y.; Hui, H.; Liang, X.; Chi, C.; Wang, K.; Wang, Z.; Tian, J. Core-Shell Gold Nanorod@Metal-Organic Framework Nanoprobes for Multimodality Diagnosis of Glioma. Adv. Mater. 2017, 29 (3), 1604381. https://doi.org/https://doi.org/10.1002/adma.201604381.
**[4]** Mao, D.; Hu, F.; Kenry; Ji, S.; Wu, W.; Ding, D.; Kong, D.; Liu, B. Metal-Organic-Framework-Assisted In Vivo Bacterial Metabolic Labeling and Precise Antibacterial Therapy. Adv. Mater. 2018, 30 (18), 1706831. https://doi.org/https://doi.org/10.1002/adma.201706831.
**[5]** Lin, S.; Liu, X.; Tan, L.; Cui, Z.; Yang, X.; Yeung, K. W. K.; Pan, H.; Wu, S. Porous Iron-Carboxylate Metal-Organic Framework: A Novel Bioplatform with Sustained Antibacterial Efficacy and Nontoxicity. ACS Appl. Mater. Interfaces 2017, 9 (22), 19248-19257. https://doi.org/10.1021/acsami.7b04810.
**[6]** Liu, X.; Zhou, Y.; Zhang, J.; Tang, L.; Luo, L.; Zeng, G. Iron Containing Metal-Organic Frameworks: Structure, Synthesis, and Applications in Environmental Remediation. ACS Appl. Mater. Interfaces 2017, 9 (24), 20255-20275. https://doi.org/10.1021/acsami.7b02563.
**[7]** Xiong, W.; Zeng, G.; Yang, Z.; Zhou, Y.; Zhang, C.; Cheng, M.; Liu, Y.; Hu, L.; Wan, J.; Zhou, C.; Xu, R.; Li, X. Adsorption of Tetracycline Antibiotics from Aqueous Solutions on Nanocomposite Multi-Walled Carbon Nanotube Functionalized MIL-53(Fe) as New Adsorbent. Sci. Total Environ. 2018, 627, 235-244. https://doi.org/10.1016/j.scitotenv.2018.01.249.
**[8]** Wang, S.; Serre, C. Toward Green Production of Water-Stable Metal-Organic Frameworks Based on High-Valence Metals with Low Toxicities. ACS Sustain. Chem. Eng. 2019, 7 (14), 11911-11927. https://doi.org/10.1021/acssuschemeng.9b01022.
**[9]** Tucker, J.; Faul, M. Industrial Research: Drug Companies Must Adopt Green Chemistry. Nature 2016, 534, 27-29. https://doi.org/10.1038/534027a.
**[10]** Chen, F.E.; Mandel, R.M.; Woods, J.J.; Lee, J.-H.; Kim, J.; Hsu, J.H.; Fuentes-Rivera, J.J.; Wilson, J.J.; Milner, P.J. Biocompatible Metal-Organic Frameworks for the Storage and Therapeutic Delivery of Hydrogen Sulfide. Chem. Sci. 2021, 12, 7848-7857, doi:10.1039/D1SC00691F.
**[11]** Diring, S.; Carné-Sánchez, A.; Zhang, J.; Ikemura, S.; Kim, C.; Inaba, H.; Kitagawa, S.; Furukawa, S. Light Responsive Metal-Organic Frameworks as Controllable CO-Releasing Cell Culture Substrates. Chem. Sci. 2017, 8, 2381-2386, doi:10.1039/C6SC04824B.
**[12]** Morris, R. E.; Wheatley, P. S. Gas Storage in Nanoporous Materials. Angew. Chemie Int. Ed. 2008, 47 (27), 4966-4981. https://doi.org/10.1002/anie.200703934.
**[13]** Carpenter, A. W.; Schoenfisch, M. H. Nitric Oxide Release: Part II. Therapeutic Applications. Chem. Soc. Rev. 2012, 41 (10), 3742-3752. https://doi.org/10.1039/C2CS15273H.
**[14]** McKinlay, A. C.; Morris, R. E.; Horcajada, P.; Férey, G.; Gref, R.; Couvreur, P.; Serre, C. BioMOFs: Metal-Organic Frameworks for Biological and Medical Applications. Angew. Chemie Int. Ed. 2010, 49 (36), 6260-6266. https://doi.org/10.1002/anie.201000048.
**[15]** Pinto, R. V.; Pinto, M. L. Chapter 14 - Nanoporous Materials: New Generation of Nitric Oxide Donors; Morbidelli, L., Bonavida, B. B. T.-T. A. of N. O. in C. and I. D., Eds.; Academic Press, 2019; pp 277-305. https://doi.org/https://doi.org/10.1016/B978-0-12-816545-4.00014-1.
**[16]** Gregg, S. T.; Yuan, Q.; Morris, R. E.; Xiao, B. Functionalised Solids Delivering Bioactive Nitric Oxide Gas for Therapeutic Applications. Mater. Today Commun. 2017, 12 (Supplement C), 95-105. https://doi.org/https://doi.org/10.1016/j.mtcomm.2017.07.007.
**[17]** Carné-Sánchez, A.; Carmona, F. J.; Kim, C.; Furukawa, S. Porous Materials as Carriers of Gasotransmitters towards Gas Biology and Therapeutic Applications. Chem. Commun. 2020, 56 (68), 9750-9766. https://doi.org/10.1039/D0CC03740K.
**[18]** McKinlay, A. C.; Xiao, B.; Wragg, D. S.; Wheatley, P. S.; Megson, I. L.; Morris, R. E. Exceptional Behavior over the Whole Adsorption-Storage-Delivery Cycle for NO in Porous Metal Organic Frameworks. J. Am. Chem. Soc. 2008, 130 (31), 10440-10444. https://doi.org/10.1021/ja801997r.
**[19]** Pinto, R. V; Wang, S.; Tavares, S. R.; Pires, J.; Antunes, F.; Vimont, A.; Clet, G.; Daturi, M.; Maurin, G.; Serre, C.; Pinto, M. L. Tuning Cellular Biological Functions Through the Controlled Release of NO from a Porous Ti-MOF. Angew. Chemie Int. Ed. 2020, 59 (13), 5135-5143. https://doi.org/10.1002/anie.201913135.
**[20]** Eubank, J. F.; Wheatley, P. S.; Lebars, G.; McKinlay, A. C.; Leclerc, H.; Horcajada, P.; Daturi, M.; Vimont, A.; Morris, R. E.; Serre, C. Porous, Rigid Metal(III)-Carboxylate Metal-Organic Frameworks for the Delivery of Nitric Oxide. APL Mater. 2014, 2 (12), 124112. https://doi.org/10.1063/1.4904069.
**[21]** Miller, S. R.; Alvarez, E.; Fradcourt, L.; Devic, T.; Wuttke, S.; Wheatley, P. S.; Steunou, N.; Bonhomme, C.; Gervais, C.; Laurencin, D.; Morris, R. E.; Vimont, A.; Daturi, M.; Horcajada, P.; Serre, C. A Rare Example of a Porous Ca-MOF for the Controlled Release of Biologically Active NO. Chem. Commun. 2013, 49 (71), 7773-7775. https://doi.org/10.1039/C3CC41987H.
**[22]** Wheatley, P. S.; McKinlay, A. C.; Morris, R. E. A Comparison of Zeolites and Metal Organic Frameworks as Storage and Delivery Vehicles for Biologically Active Nitric Oxide. Stud. Surf. Sci. Catal. 2008, 174, 441-446. https://doi.org/10.1016/S0167-2991(08)80236-4.
**[23]** Xiao, B.; Wheatley, P. S.; Zhao, X.; Fletcher, A. J.; Fox, S.; Rossi, A. G.; Megson, I. L.; Bordiga, S.; Regli, L.; Thomas, K. M.; Morris, R. E. High-Capacity Hydrogen and Nitric Oxide Adsorption and Storage in a Metal-Organic Framework. J. Am. Chem. Soc. 2007, 129 (5), 1203-1209. https://doi.org/10.1021/ja066098k.
**[24]** Hinks, N. J.; McKinlay, A. C.; Xiao, B.; Wheatley, P. S.; Morris, R. E. Metal Organic Frameworks as NO Delivery Materials for Biological Applications. Microporous Mesoporous Mater. 2010, 129 (3), 330-334. https://doi.org/10.1016/j.micromeso.2009.04.031.
**[25]** Rouquérol, F.; Rouquérol, J.; Sing, K. Adsorption by powders and porous solids; Academic Press: San Diego, 1999**.**
**[26]** WO2009/123484
**[27]** M. Rose et al. Adv. Eng. Mater. 2011, 13, 356-360.
**[28]** R. Ostermann at al. Chem. Commun. 2011, 47, 442-444.
**[29]** J. Ren et al. Int. J. Hydrogen Energy 2015, 40, 9382-9387 .
**[30]** M.R. Khan et al. J. Mater. Eng. Perform. 2016, 25, 1276-1283.
**[31]** Valekar et al., RSC Adv, 2017
**[32]** Q. Ren, et al.," Chem. Eng. J., , 2015
**[33]** Gkaniatsou et al., Nano Energy, 2020
**[34]** McKinlay, A.C.; Eubank, J.F.; Wuttke, S.; Xiao, B.; Wheatley, P.S.; Bazin, P.; Lavalley, J.-C.; Daturi, M.; Vimont, A.; De Weireld, G.; et al. Nitric Oxide Adsorption and Delivery in Flexible MIL-88(Fe) Metal-Organic Frameworks. Chem. Mater. 2013, 25, 1592-1599, doi:10.1021/cm304037x.
**[35]** Feelisch, M.; Kubitzek, D.; Werringloer, J. The Oxyhemoglobin Assay. In Methods in Nitric Oxide Research; Feelisch, M., Stamler, J.S., Eds.; John Wiley & Sons, Ltd: New York, 1996; pp. 455-478.
**[36]** Lin, Z.; Pinto, R. V; Henriques, C.; Antunes, F.; Pires, J.; Pinto, M.L.; Rocha, J. Improved Therapeutic Nitric Oxide Delivery by Microporous Cu-Bearing Titanosilicate. Microporous Mesoporous Mater. 2021, 111154, doi:https://doi.org/10.1016/j.micromeso.2021.111154.

## Claims

1. An ultra-microporous crystalline metal organic framework (MOF)solid for use in the controlled release of the Lewis-based gas in wounds, preferably by topical application,
the ultra-microporous crystalline metal organic framework solid comprising a three-dimensional succession of units corresponding to the following formula (I):
MₘXₙL•yH₂O (I)
wherein
- M independently represents a metal chosen from Fe, Al, V, Mn, Ti, Zr and mixtures thereof, M being in oxidation state III or IV, preferably a metal chosen from Fe, Al and Ti, and more preferably Fe,
- L represents a bisphosphonic acid ligand,
- X represents an anion, preferably chosen from O²⁻, HO⁻, F⁻, SO₄²⁻, HSO₄⁻, H₂PO₄⁻, HPO₄²⁻ and PO₄³⁻, preferably chosen from O²⁻ and HO⁻,
- m is an integer from 1 to 4
- n = 0 or 1, and
- y = 0, 1 or 2,
wherein said ultra-microporous crystalline metal organic framework solid is loaded with at least one Lewis-based gas, at least a part of which is coordinated with the metal M, and
wherein the at least one Lewis-based gas is chosen from NO, CO and H₂S, preferably NO..

2. The MOF for use according to claim 1, wherein the ligand L is a bisphosphonic acid ligand comprising from 4 to 12 carbon atoms, preferably chosen from the bisphosphonic acid ligands of formula (II):
HPO₃-CH₂-R-CH₂-PO₃ (II),
in which
R is an optionally substituted C₄ to C₆ cyclic or heterocyclic moiety, optionally comprising heteroatoms such as N, optionally aromatic.

3. The MOF for use according to any of the preceding claims, wherein the ligand L is chosen from p-xylylenebisphosphonic acid ligand, 1,4-bis(phosphomethyl)piperazine acid ligand, 1,4-bis(phosphomethyl)-2-methylpiperazine acid ligand and mixture thereof.

4. The MOF for use according to any of the preceding claims, wherein the MOF has an average pore diameter size from 0.3 to 0.5 nm.

5. The MOF for use according to any of the preceding claims, wherein the solid has a gas loading capacity of 0.5 to 4 mmol of Lewis-based gas per gram of dry MOF, preferably between 1 and 2 mmol/g.

6. The MOF for use according to any of preceding claims, wherein the MOF has a three-dimensional succession of units chosen from:
-
M(HPO₃-CH₂-C₆H₄-CH₂-PO₃)Xₙ•H₂O,
M being in oxidation state III or IV,
with M preferably being Fe, Al or Ti and when M = Al or Fe, n = 0 and when M = Ti, n = 1;
-
M(HPO₃-CH₂-C₄H₈N₂-CH₂-PO₃)X ₙ•H₂O,
M being in oxidation state III or IV,
with M preferably being Fe, Al or Ti and when M = Al or Fe, n = 0 and when M = Ti, n = 1

7. The MOF for use according to any of preceding claims, wherein at least 30% of the adsorbed amount of the Lewis-based gas coordinates with M.

8. The MOF for use according to any of the preceding claims, wherein the controlled release of the Lewis-based gas is a slow release of at least more than 24 hours, preferably from 24 hours to 72 hours.

## Patentansprüche

1. Ultramikroporöser kristalliner metallorganischer Gerüst-(MOF-)Feststoff zur Verwendung bei der kontrollierten Freisetzung des Lewis-basierten Gases in Wunden, vorzugsweise durch topische Anwendung,
wobei der ultramikroporöse kristalline metallorganische Gerüstfeststoff eine dreidimensionale Abfolge von Einheiten umfasst, die folgender Formel entsprechen (I):
MₘXₙL•yH2 O (I)
wobei
- M unabhängig ein Metall darstellt, das aus Fe, Al, V, Mn, Ti, Zr und Gemischen davon ausgewählt ist, wobei M im Oxidationszustand III oder IV ist, vorzugsweise ein Metall, ausgewählt aus Fe, Al und Ti, und noch bevorzugter Fe,
- L einen bisphosphonischen Säureligand darstellt,
- X ein Anion darstellt, vorzugsweise ausgewählt aus O²⁻, HO⁻, F⁻, SO₄²⁻, HSO₄⁻, H₂ PO₄⁻, HPO₄²⁻ und PO₄³⁻, vorzugsweise ausgewählt aus O²⁻ und HO⁻,
- m eine ganze Zahl von 1 bis 4 ist
- n = 0 oder 1 und
- y = 0, 1 oder 2,
wobei der ultramikroporöse kristalline metallorganische Gerüstfeststoff mit mindestens einem Lewis-basierten Gas beladen ist, von dem mindestens ein Teil mit dem Metall M koordiniert ist, und
wobei das mindestens eine Lewis-basierte Gas aus NO, CO und H₂S, vorzugsweise NO, ausgewählt ist.

2. MOF zur Verwendung nach Anspruch 1, wobei der Ligand L ein bisphosphonischer Säureligand ist, der 4 bis 12 Kohlenstoffatome umfasst, vorzugsweise ausgewählt aus den bisphosphonischen Säureliganden der Formel (II):
HPO₃-CH₂-R-CH₂-PO₃ (II),
wobei:
R ein optional substituierter C₄ bis C₆ zyklischer oder heterozyklischer Anteil ist, optional umfassend Heteroatome wie N, optional aromatisch.

3. MOF zur Verwendung nach einem der vorhergehenden Ansprüche, wobei der Ligand L aus p-Xylylenbisphosphonsäure-Ligand, 1,4-bis(Phosphomethyl)piperazinsäure-Ligand, 1,4-bis(Phosphomethyl)-2-methylpiperazinsäure-Ligand und einem Gemisch davon ausgewählt ist.

4. MOF zur Verwendung nach einem der vorhergehenden Ansprüche, wobei das MOF einen durchschnittlichen Porendurchmesser von 0,3 bis 0,5 nm aufweist.

5. MOF zur Verwendung nach einem der vorhergehenden Ansprüche, wobei der Feststoff eine Gasbelastbarkeit von 0,5 bis 4 mmol Lewis-basiertes Gas pro Gramm trockenes MOF aufweist, vorzugsweise zwischen 1 und 2 mmol/g.

6. MOF zur Verwendung nach einem der vorhergehenden Ansprüche, wobei das MOF eine dreidimensionale Abfolge von Einheiten aufweist, die ausgewählt sind aus:
-
M(HPO₃-CH₂-C₆H₄-CH₂-PO₃)Xₙ•H₂O,
wobei M im Oxidationszustand III oder IV ist,
wobei M vorzugsweise Fe, Al oder Ti ist und wenn M = Al oder Fe, n = 0 und wenn M = Ti, n = 1;
-
M(HPO₃-CH₂-C₄H₈N₂-CH₂-PO₃)Xₙ•H₂O,
wobei M im Oxidationszustand III oder IV ist,
wobei M bevorzugt Fe, Al oder Ti ist und wenn M = Al oder Fe, n = 0 und wenn M = Ti, n = 1

7. MOF zur Verwendung nach einem der vorhergehenden Ansprüche, wobei mindestens 30 % der adsorbierten Menge des Lewis-basierten Gases mit M koordiniert sind.

8. MOF zur Verwendung nach einem der vorhergehenden Ansprüche, wobei die kontrollierte Freisetzung des Lewis-basierten Gases eine langsame Freisetzung von mindestens mehr als 24 Stunden, vorzugsweise von 24 Stunden bis 72 Stunden, ist.

## Revendications

1. Solide de type réseau organométallique (MOF) cristallin ultra-microporeux pour une utilisation dans la libération contrôlée du gaz de type base de Lewis dans des plaies, de préférence par application topique,
le solide de type réseau organométallique cristallin ultra-microporeux comportant une succession tridimensionnelle de motifs correspondant à la formule (I) suivante :
MₘXₙL•yH₂O (I)
dans laquelle
- M représente indépendamment un métal choisi parmi Fe, Al, V, Mn, Ti, Zr et les mélanges de ceux-ci, M étant à l'état d'oxydation III ou IV,
de préférence un métal choisi parmi Fe, Al et Ti, et plus préférentiellement Fe,
- L représente un ligand de type acide bisphosphonique,
- X représente un anion, de préférence choisi parmi O²⁻, HO⁻, F⁻, SO₄²⁻, HSO₄⁻, H₂PO₄⁻, HPO₄²⁻ et PO₄³⁻, de préférence choisi parmi O²⁻ et HO⁻,
- m est un nombre entier de 1 à 4 ;
- n = 0 ou 1, et
- y = 0, 1 ou 2,
dans lequel ledit solide de type réseau organométallique cristallin ultra-microporeux est chargé avec au moins un gaz de type base de Lewis, dont au moins une partie est coordonnée au métal M, et
dans lequel l'au moins un gaz de type base de Lewis est choisi parmi NO, CO et H₂S, de préférence NO.

2. MOF pour une utilisation selon la revendication 1, dans lequel le ligand L est un ligand de type acide bisphosphonique comportant de 4 à 12 atomes de carbone, de préférence choisi parmi les ligands de type acide bisphosphonique de formule (II) :
HPO₃-CH₂-R-CH₂-PO₃ (II),
dans laquelle
R est un fragment cyclique ou hétérocyclique en C₄ à C₆ facultativement substitué, comportant facultativement des hétéroatomes tels que N, facultativement aromatique.

3. MOF pour une utilisation selon l'une quelconque des revendications précédentes, dans lequel le ligand L est choisi parmi le ligand acide p-xylylènebisphosphonique, le ligand acide 1,4-bis(phosphométhyl)pipérazine, le ligand acide 1,4-bis(phosphométhyl)-2-méthylpipérazine et un mélange de ceux-ci.

4. MOF pour une utilisation selon l'une quelconque des revendications précédentes, dans lequel le MOF présente un diamètre moyen de pore de 0,3 à 0,5 nm.

5. MOF pour une utilisation selon l'une quelconque des revendications précédentes, dans lequel le solide présente une capacité de chargement en gaz de 0,5 à 4 mmol de gaz de type base de Lewis par gramme de MOF sec, de préférence entre 1 et 2 mmol/g.

6. MOF pour une utilisation selon l'une quelconque des revendications précédentes, dans lequel le MOF présente une succession tridimensionnelle de motifs choisis parmi :
-
M(HPO₃-CH₂-C₆H₄-CH₂-PO₃)Xₙ•H₂O,
M étant à l'état d'oxydation III ou IV,
M étant de préférence Fe, Al ou Ti et lorsque M = Al ou Fe, n = 0 et lorsque M = Ti, n = 1 ;
-
M(HPO₃-CH₂-C₄H₈N₂-CH₂-PO₃)Xₙ•H₂O,
M étant à l'état d'oxydation III ou IV,
M étant de préférence Fe, Al ou Ti et lorsque M = Al ou Fe, n = 0 et lorsque M = Ti, n = 1

7. MOF pour une utilisation selon l'une quelconque des revendications précédentes, dans lequel au moins 30 % de la quantité adsorbée du gaz de type base de Lewis se coordonnent à M.

8. MOF pour une utilisation selon l'une quelconque des revendications précédentes, dans lequel la libération contrôlée du gaz de type base de Lewis est une libération lente d'au moins plus de 24 heures, de préférence de 24 heures à 72 heures.
